# EUROPEAN PATENT SPECIFICATION

(11) **EP 2 139 479 B1**
(45) Date of publication and mention of the grant of the patent: **12.12.2012**
(21) Application number: 08715618.8
(22) Date of filing: 14.03.2008
(51) Int. Cl.: A61K 31/451, C07D 211/20, A61P 25/24, A61P 25/28

(54) **4-[2-(4-METHYLPHENYLSULFANYL)PHENYL]PIPERIDINE WITH COMBINED SEROTONIN AND NOREPINEPHRINE REUPTAKE INHIBITION FOR THE TREATMENT OF ADHD, MELANCHOLIA, TREATMENT RESISTENT DEPRESSION OR RESIDUAL SYMPTOMS IN DEPRESSION**
4-[2-(4-METHYLPHENYLSULFANYL)PHENYL]PIPERIDIN MIT KOMBINIERTER SEROTONIN- UND NOREPINEPHRIN-WIEDERAUFNAHMEHEMMUNG ZUR BEHANDLUNG VON ADHD, MELANCHOLIE, THERAPIEREFRAKTÄRER DEPRESSION ODER DEPRESSIVEN RESTSYMPTOMEN
4-[2-(4-METHYLPHENYLSULFANYL)PHENYL]PIPERIDINE AVEC INHIBITION COMBINÉ DU RECAPTAGE DE LA SEROTONINE ET DE LA NOREPINEPHRINE POUR LE TRAITEMENT DE L'ADHD, DE LA MÉLANCOLIE, DE LA DEPRESSION RESISTENT AU TRAITEMENT OU DE SYMPTÔMES RESIDUELLES EN DEPRESSION

(30) Priority: 20.03.2007 DK 200700423; 15.06.2007 WO PCT/DK2007/050076
(43) Date of publication of application: 06.01.2010
(73) Proprietor: H. Lundbeck A/S, 2500 Valby (DK)
(72) Inventor: STENSBØL, Tine, Bryan, DK-3500 Værløse (DK); MILLER, Silke, Monroe, New York 10950 (US)
(74) Representative: Conrad, Lars Sparre
(86) International application number: PCT/DK2008/050064
(87) International publication number: WO 2008/113360

(56) References cited:
- WO-A-03/029232
- WO-A-2006/007843
- WO-A-2007/144006

## Description

### Background

The compound 4-[2-(4-methylphenylsulfanyl)phenyl]piperidine is disclosed in the international patent application WO 03/029232. The compound is said to be an inhibitor of the serotonin transporter, to have affinity for the serotonin receptor 2C (5-HT_{2C}), and as such be useful if the treatment of mood disorders, such as major depression and anxiety.

WO 2006/007843 discloses certain indolylsulfanylaryl amines useful in the treatment of ADHD

As shown in the examples, however, said compound is endowed with a broader pharmacological profile, which makes the compound useful in the treatment of other diseases as well - treatments for which there is a desire. This pharmacological profile is also disclosed in WO 07/144006 together with the use of said compound in the treatment of additional diseases.

### Summary of the invention

In one embodiment, the invention relates to 4-[2-(4-methylphenylsulfanyl)-phenyl]piperidine and acid additions salts thereof (compound I) for use in the treatment of ADHD

### Figures

Figure 1: X-ray diffraction pattern of the HBr addition salt of compound I
Figure 2: X-ray diffraction pattern of the HBr addition salt solvate of compound I
Figure 3: X-ray diffraction pattern of the palmitic acid addition salt of compound I
Figure 4: X-ray diffraction pattern of the DL-lactic acid addition salt of compound I
Figure 5: X-ray diffraction pattern of the adipic acid addition salt (1:1) of compound I (α+β form)
Figure 6: X-ray diffraction pattern of the adipic acid addition salt (2:1) of compound I
Figure 7: X-ray diffraction pattern of the fumaric acid addition salt (1:1) of compound I
Figure 8: X-ray diffraction pattern of the glutaric acid addition salt (1:1) of compound I
Figure 9: X-ray diffraction pattern of the malonic acid addition salt (1:1) of compound I, α-form
Figure 10: X-ray diffraction pattern of the malonic acid addition salt of compound I, β-form
Figure 11: X-ray diffraction pattern of the oxalic acid addition salt (1:1) of compound I
Figure 12: X-ray diffraction pattern of the sebacoinic acid addition salt (2:1) of compound I
Figure 13: X-ray diffraction pattern of the succinic acid addition salt (2:1) of compound I
Figure 14: X-ray diffraction pattern of the L-malic acid addition salt (1:1) of compound I, α-form
Figure 15: X-ray diffraction pattern of the L-malic acid addition salt (1:1) of compound I, β-form
Figure 16: X-ray diffraction pattern of the D-tartaric acid addition salt (1:1) of compound I
Figure 17: X-ray diffraction pattern of the L-aspartic acid addition salt (1:1) of compound I in mixture with L-aspartic acid
Figure 18: X-ray diffraction pattern of the L-aspartic acid addition salt hydrate (1:1) of compound I in mixture with L-aspartic acid
Figure 19: X-ray diffraction pattern of the glutamic acid addition salt (1:1) of compound I in mixture with glutamic acid monohydrate
Figure 20: X-ray diffraction pattern of the citric acid addition salt (2:1) of compound I
Figure 21: X-ray diffraction pattern of the HCl acid addition salt of compound I
Figure 22: X-ray diffraction pattern of the phosphoric acid addition salt (1:1) of compound I
Figure 23: Dopamine levels in prefrontal cortex upon administration of compound I.
Figure 24: Acetylcholine levels in prefrontal cortex upon administration of compound I.
Figure 25a+b: Acetylcholine levels in the prefrontal cortex and ventral hippocampus upon administration of compound I.
Figure 26: Effect of compound I on attention-deficits and impulsivity I SHR rats.

### Detailed description of the invention

The present invention relates to the use of compound I, which is 4-[2-(4-methylphenylsulfanyl)-phenyl]piperidine and pharmaceutically acceptable salts thereof. The structure of 4-[2-(4-methylphenylsulfanyl)-phenyl]piperidine is

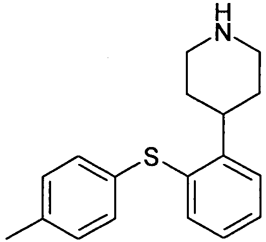

The pharmacological profile of compound I is depicted in the examples, but can be summarised as follows. The compound inhibits the serotonin and norepinephrine reuptake; it inhibits the serotonin receptors 2A, 2C and 3; and it inhibits the α-1 adrenergic receptor.

In one embodiment, said acid addition salts are salts of acids that are nontoxic. Said salts include salts made from organic acids, such as maleic, fumaric, benzoic, ascorbic, succinic, oxalic, bis-methylenesalicylic, methanesulfonic, ethanedisulfonic, acetic, propionic, tartaric, salicylic, citric, gluconic, lactic, malic, malonic, mandelic, cinnamic, citraconic, aspartic, stearic, palmitic, itaconic, glycolic, p-aminobenzoic, glutamic, benzenesulfonic, theophylline acetic acids, as well as the 8-halotheophyllines, for example 8-bromotheophylline. Said salts may also be made from inorganic salts, such as hydrobromic, sulfuric, sulfamic, phosphoric and nitric acids.

In one embodiment, compound I is the HBr addition salt

In one embodiment, compound I is the DL-lactic acid addition salt, and in particular the 1:1 salt.

In one embodiment, compound I is the L-aspartic acid addition salt, and in particular the 1:1 salt.

In one embodiment, compound I is the glutamic acid addition salt, and in particular the 1:1 salt.

In one embodiment, compound I is the glutaric acid addition salt, and in particular the 1:1 salt.

In one embodiment, compound I is the malonic acid addition salt, and in particular the 1:1 salt that is found to exist in two polymorphic modifications α and of which the β form is believed to be the most stable based on a lower solubility.

In one embodiment, compound I is in a purified form. The term "purified form" is intended to indicate that the compound is essentially free of other compounds or other forms, i.e. polymorphs of said compound, as the case may be.

Oral dosage forms, and in particular tablets and capsules, are often preferred by the patients and the medical practitioner due to the ease of administration and the consequently better compliance. For tablets and capsules, it is preferable that the active ingredients are crystalline. In one embodiment, compound I is crystalline.

Crystals used in the present invention may exist as solvates, i.e. crystals wherein solvent molecules form part of the crystal structure. The solvate may be formed from water, in which case the solvates are often referred to as hydrates. Alternatively, the solvates may be formed from other solvents, such as e.g. ethanol, acetone, or ethyl acetate. The exact amount of solvate often depends on the conditions. For instance, hydrates will typically loose water as the temperature is increased or as the relative humidity is decreased. Compounds, which do not change or which change only little when conditions, such as e.g. humidity change are generally regarded as better suited for pharmaceutical formulations. It is noted that the HBr acid addition salt does not form hydrates when precipitated from water whereas compounds such as the succinate, malate and tatrate acid addition salts do.

Some compounds are hygroscopic, i.e. they absorb water when exposed to humidity. Hygroscopicity is generally regarded as an undesired property for compounds, which are to be presented in a pharmaceutical formulation, in particular in a dry formulations, such as tablets or capsules. In one embodiment, the invention provides crystals with low hygroscopicity.

For oral dosage forms using crystalline active ingredients it is also beneficial if said crystals are well-defined. In the present context, the term "well-defined" in particular means that the stoichiometry is well-defined, i.e. that the ratio between the ions forming the salt is the ratio between small integers, such as 1:1, 1:2, 2:1, 1:1:1, etc. In one embodiment, the compounds of the present invention are well-defined crystals.

The solubility of an active ingredient is also of significance for the choice of dosage form as it may have a direct impact on bio-availability. For oral dosage forms, a higher solubility of the active ingredient is generally believed to be beneficial as it increases the bio-availability. Some patients, e.g. elderly patients may have difficulties swallowing tablets, and oral drop solutions may be a suitable alternative avoiding the need for swallowing tablets. In order to limit the volume of an oral drop solution, it is necessary to have a high concentration of the active ingredient in the solution, which again requires a high solubility of the compound. As shown in table 3, DL-lactic acid, L-aspartic acid, glutamic acid, glutaric acid and malonic acid addition salts have exceptionally high solubility.

Crystal forms impact the filtration and processing properties of a compound. Needle formed crystals tend to be more difficult to handle in a production environment as filtration becomes more difficult and time consuming. The exact crystal form of a given salt may depend e.g. on the conditions under which the salt was precipitated. The HBr acid addition salt of compound I grows needle-shaped, solvated crystals when precipitated from ethanol, acetic acid and propanol, but crystals of a non-hydrated form, which are not needle-shaped, when HBr addition salt is precipitated from water, providing superior filtration properties.

Table 3 also depicts the Resulting pH, i.e. the pH in the saturated solution of the salt. This property is of importance because moisture can never be completely avoided during storage and the accumulation of moisture will give rise to a pH decrease in or on a tablet comprising a low Resulting pH salt, which may decrease shell life. Moreover, a salt with a low resulting pH may give rise to corrosion of process equipment if tablets are made by wet granulation. The data in table 3 suggest that the HBr, HCl and adipic acid addition salts may be superior in this respect.

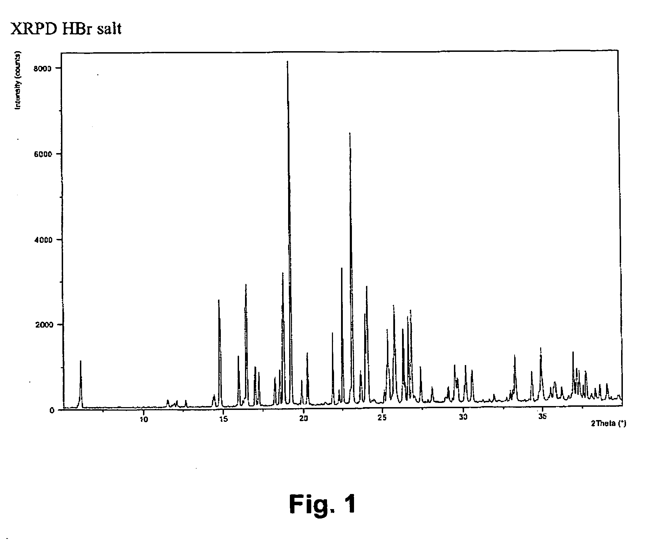

In one embodiment, compound I is the HBr addition salt in a crystalline form, in particular in a purified form. In a further embodiment, said HBr salt has peaks in an X-ray powder diffractogram (XRPD) at approximately 6.08°, 14.81°, 19.26° and 25.38°2θ, and in particular said HBr salt has an XRPD as depicted in figure 1.

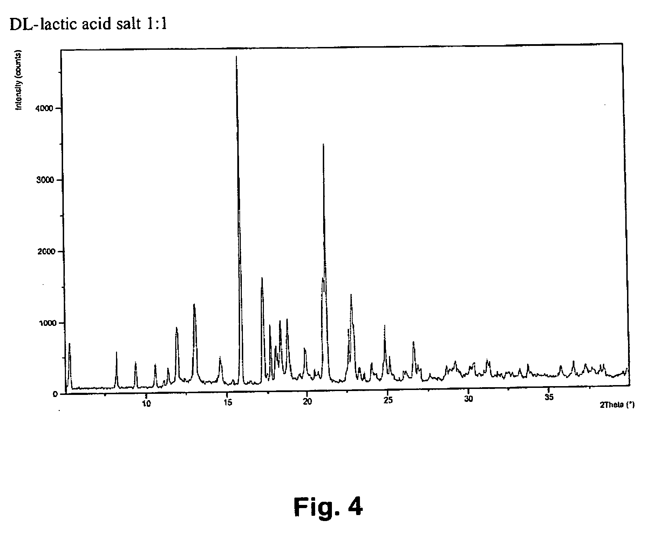

In one embodiment, compound I is the DL-lactic acid addition salt (1:1) in a crystalline form, in particular in a purified form. In a further embodiment, said DL-lactic acid addition salt has peaks in a XRPD at approximately 5.30°, 8.81°, 9.44° and 17.24°2θ, and in particular said DL lactic acid addition salt has an XRPD as depicted in figure 4.

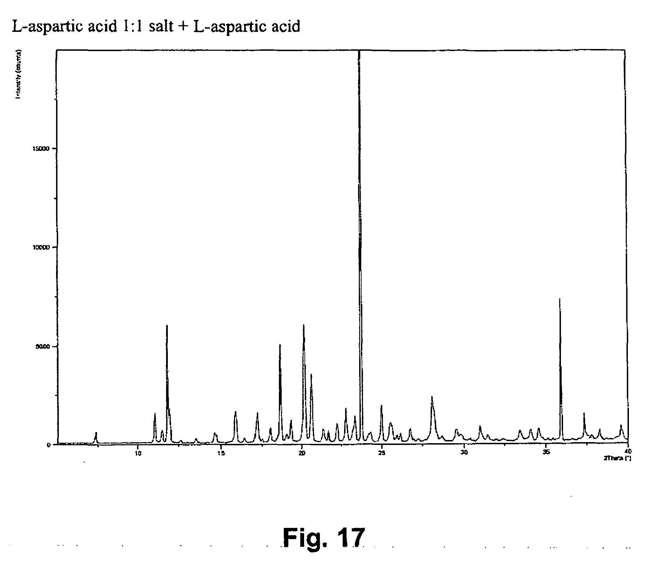

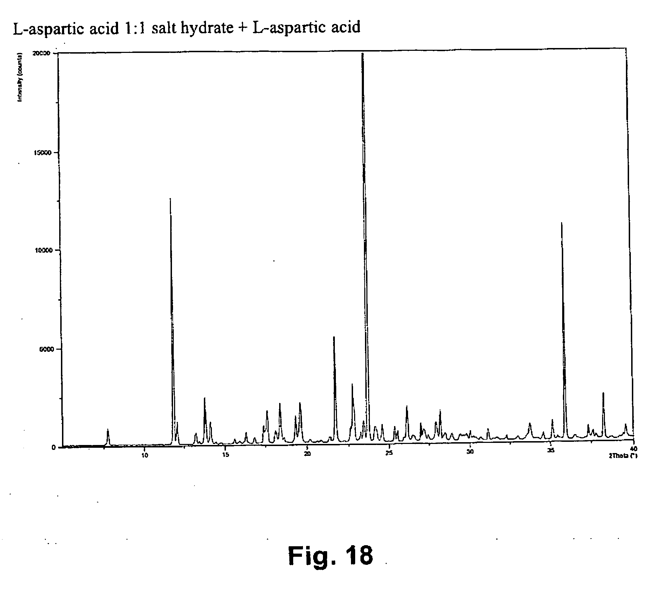

In one embodiment, compound I is the L-aspartic acid addition salt (1:1) in a crystalline form, in particular in a purified form. In a further embodiment, said L-aspartic acid addition salt is unsolvated and has peaks in a XRPD at approximately 11.05°, 20.16°, 20.60°, 25.00°2θ, and in particular said L-aspartic salt, when mixed with L-aspartic acid, has an XRPD as depicted in figure 17. In one embodiment, said L-aspartic acid addition salt is a hydrate, in particular in a purified form. In a further embodiment, said L-aspartic acid addition salt hydrate has peaks in a XRPD at approximately 7.80°, 13.80°, 14.10°, 19.63°28, and in particular said L-aspartic addition salt hydrate, when mixed with L-aspartic acid, has an XRPD as depicted in figure 18.

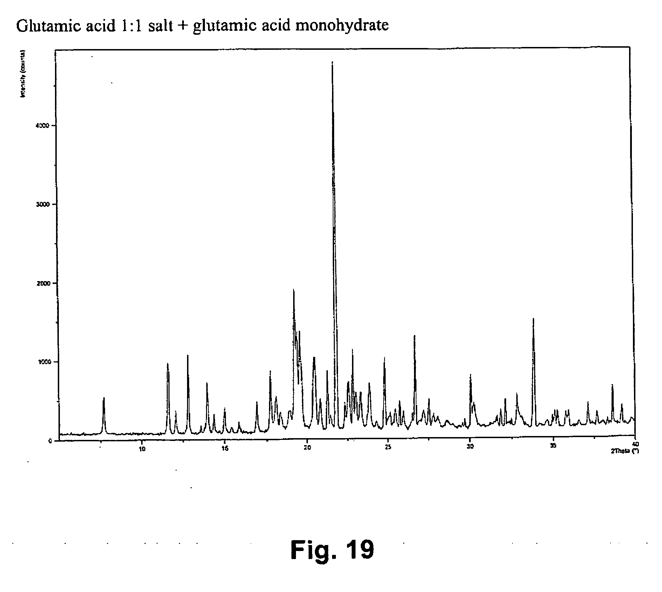

In one embodiment, compound I is the glutamic acid addition salt (1:1) in a crystalline form, in particular in a purified form. In a further embodiment, said glutamic acid addition salt has peaks in a XRPD at approximately 7.71°, 14.01°, 19.26°, 22.57°2θ, and in particular said glutamic acid salt, when mixed with glutamic acid monohydrate, has an XRPD as depicted in figure 19.

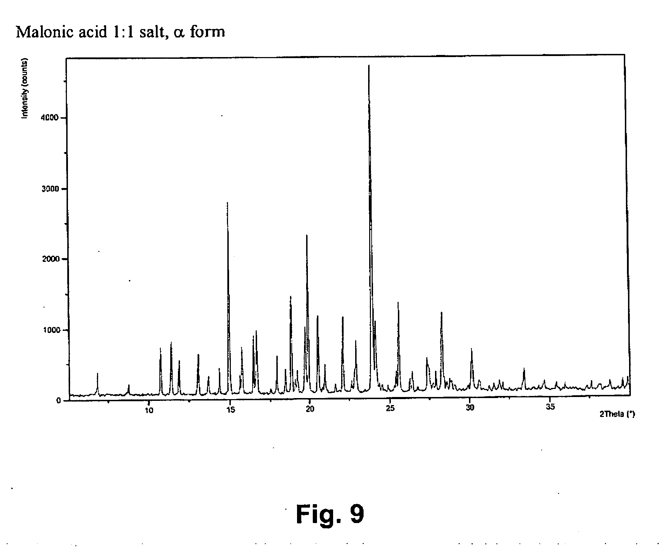

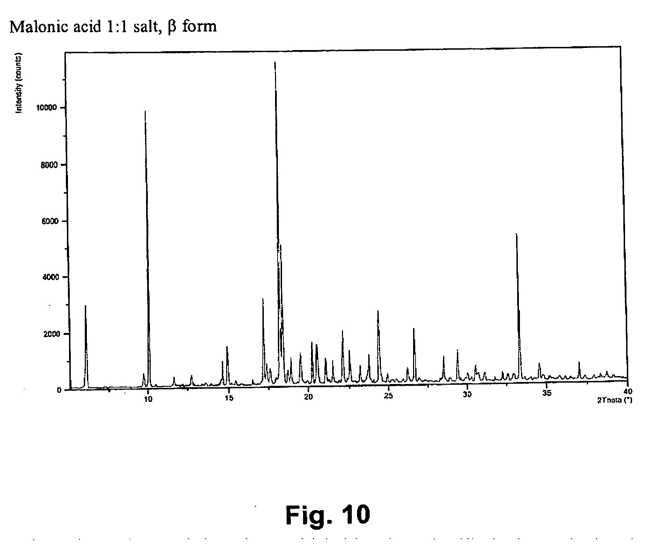

In one embodiment, compound I is the malonic acid addition salt (1:1) in a crystalline form, in particular in a purified form. In a further embodiment, said malonic acid addition salt is the α-form and has peaks in a XRPD at approximately 10.77°, 16.70°, 19.93°, 24.01°2θ, or said malonic acid addition salt is the β-form and has peaks in a XRPD at approximately 6.08°, 10.11°, 18.25°, 20.26°2θ and in particular said malonic acid addition salt has an XRPD as depicted in figure 9 or 10.

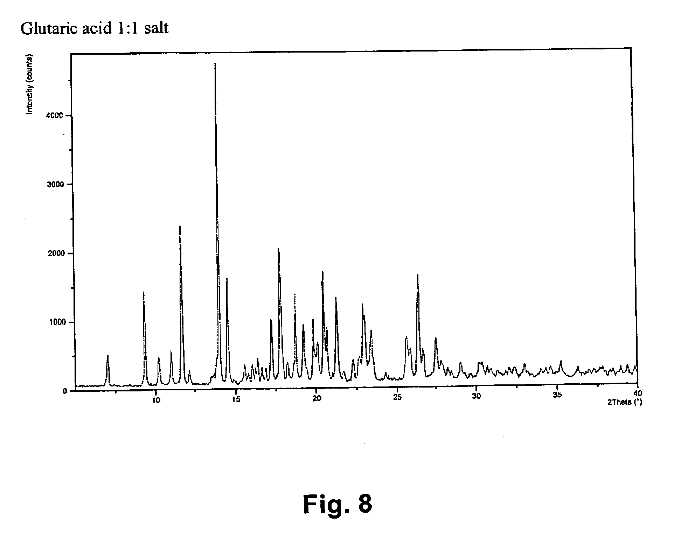

In one embodiment, compound I is the glutaric acid addition salt (1:1) in a crystalline form, in particular in a purified form. In a further embodiment, said glutaric acid addition salt has peaks in a XRPD at approximately 9.39°, 11.70°, 14.05°, and 14.58°2θ, and in particular said glutaric acid addition salt has an XRPD as depicted in figure 8.

The unique pharmacological profile of compound I makes it suitable for the treatment of diseases beyond those disclosed in WO 03/029232. 5-HT_{2C} receptors are located e.g. on dopaminergic neurons where activation exerts a tonic inhibitory influence on the dopamine release, and 5-HT_{2C} antagonists will effect an increase in the dopamine level. Data presented in example 2E show that compound I does, in deed, bring about a dose dependent increase in the extra cellular dopamine levels in the prefrontal cortex. On this background it may be hypothesized that 5-HT_{2C} antagonists are particular well-suited for the treatment of depression which is refractory to the treatment with selective serotonin reuptake inhibitors [Psychopharmacol. Bull., 39, 147-166, 2006]. This hypothesis finds support in several clinical studies showing a combination of mirtazipine and SSRI to be superior to SSRI alone for the treatment of depressed patients with an inadequate clinical response (treatment resistant depression, TRD, or refractory depression) [Psychother. Psychosom., 75, 139-153, 2006]. Mirtazapine is also a 5-HT₂ and a 5-HT₃ antagonist, which indicates that compounds exerting serotonin reuptake inhibition in combination with 5-HT₂ and 5-HT₃ antagonism, such as compound I is useful for the treatment of TRD, i.e. will increase the remission rate for patients suffering from treatment resistant depression.

Data presented in example 2F and 2G show that compound I brings about an increase in the extracellular level of acetylcholine in the prefrontal cortex and ventral hippocampus. There is longstanding clinical evidence that increasing the acetylcholine levels in the brain is a way to treat Alzheimer's disease and cognitive impairment in general, cf. the use of acetylcholine esterase inhibitors in the treatment of Alzheimer's disease. On this background, compounds of the present invention are believed to be useful in the treatment of Alzheimer's disease and cognitive impairment, and also mood disorders, such as depression associated with Alzheimer's disease and cognitive impairment.

A segment of depressed patients will respond to treatment with antidepressants, such as e.g. SSRI's in the sense that they will improve on clinically relevant depression scales, such as MADRD and HAMD, but where other symptoms, such as sleep disturbances and cognitive impairment remain. In the present context, these patients are referred to as partial responders. Due to the above-discussed effects on the acetylcholine levels, the compounds of the present invention are expected to be useful in the treatment of the cognitive impairment in addition to the depression. Clinical studies have shown that the compound prazosin, which is an α-1 adrenergic receptor antagonist reduces sleep disturbances [Biol. Psychiatry, 61, 928-934, 2007]. Moreover, the 5-HT_{2A} and 5-HT_{2C} antagonism of the compounds of the present invention are also believed to have a sedative, sleep-improving effect [Neuropharmacol, 33, 467-471, 1994] wherefore compound I is useful for the treatment of partial responders, or rephrased that treatment of depressed patients with compound I will reduce the fraction of partial responders.

Attention deficit hyperactivity disorder (ADHD) is one of the most common neurobehavoioral disorders. ADHD is characterised by the presence of a triad of social and communicative impairments with restricted, repetitive or stereotyped behaviours. ADHD usually starts in childhood or adolescence, but symptoms may continue into adulthood. Atomoxetine is currently the only nonstimulant approved by FDA for the treatment of ADHD [Drugs, 64, 205-222, 2004]. Atomoxetine is a norepinephrine reuptake inhibitor, which also brings about in increase in the dopamine level in the pre-frontal cortex. It has been suggested that the increase in the level of said neurotransmitters mediates the therapeutic effect of atomoxetine in the treatment of ADHD [Eur.Neuropsychopharmacol., 12, suppl. 3, 418, 2002]. This supports the notion that compound I may be used in the treatment of ADHD. In addition, compounds of the present invention may have a sedative effect due to the α-1 adrenergic receptor and 5-HT₂ antagonism discussed above, which is beneficial in the treatment of ADHD. As shown in example 3 studies in rats show that compound I reduces hyperactivity, impulsiveness and attention deficits.

Melancholia is a particular subtype of depression often connected to severe depression; this type of depression is also referred to as melancholic depression. Melancholia is associated with anxiety, dread of the future, insomnia, and loss of appetite. Compounds that inhibit both the serotonin and the norepinephrine reuptake, such as e.g. venlafaxine, have been shown to be particular effective in the treatment of patients with severe depression and melancholia [Depres. Anxiety, 12, 50-54, 2000]. As discussed above, compounds exerting 5-HT_{2C} antagonism increase the dopamine level, wherefore such compounds would be expected to be effective in the treatment of melancholia [Psychpharm. Bull., 39, 147-166, 2006]. Additionally, the α-1 adrenergic receptor and 5-HT₂ antagonism of the compounds of the present invention is expected to help normalise sleep, wherefore said compounds are useful in the treatment of melancholia.

In an embodiment, the compound of the invention is administered in an amount of about 0.001 to about 100 mg/kg body weight per day.

A typical oral dosage is in the range of from about 0.001 to about 100 mg/kg body weight per day, preferably from about 0.01 to about 50 mg/kg body weight per day, administered in one or more dosages such as 1 to 3 dosages. The exact dosage will depend upon the frequency and mode of administration, the sex, age, weight and general condition of the subject treated, the nature and severity of the condition treated and any concomitant diseases to be treated and other factors evident to those skilled in the art.

A typical oral dosage for adults is in the range of 1-100 mg/day of a compound of the present invention, such as 1-30 mg/day, 5-25 mg/day or 5-60 mg/day. This may typically be achieved by the administration of 0.1- 60 mg, such as 0.1-50 mg, 1-25 mg, 1-35 mg, such as 1, 5, 10, 15, 20, 25, 30, 35, 40, 45, 50, 55 or 60 mg of compound I once or twice daily.

A "therapeutically effective amount" of a compound as used herein means an amount sufficient to cure, alleviate or partially arrest the clinical manifestations of a given disease and its complications in a therapeutic intervention comprising the administration of said compound. An amount adequate to accomplish this is defined as "therapeutically effective amount". The term also includes amounts sufficient to cure, alleviate or partially arrest the clinical manifestations of a given disease and its complications in a treatment comprising the administration of said compound. Effective amounts for each purpose will depend on the severity of the disease or injury as well as the weight and general state of the subject. It will be understood that determining an appropriate dosage may be achieved using routine experimentation, by constructing a matrix of values and testing different points in the matrix, which is all within the ordinary skills of a trained physician.

The term "treatment" and "treating" as used herein means the management and care of a patient for the purpose of combating a condition, such as a disease or a disorder. The term is intended to include the full spectrum of treatments for a given condition from which the patient is suffering, such as administration of the active compound to alleviate the symptoms or complications, to delay the progression of the disease, disorder or condition, to alleviate or relief the symptoms and complications, and/or to cure or eliminate the disease, disorder or condition as well as to prevent the condition, wherein prevention is to be understood as the management and care of a patient for the purpose of combating the disease, condition, or disorder and includes the administration of the active compounds to prevent the onset of the symptoms or complications. Nonetheless, prophylactic (preventive) and therapeutic (curative) treatment are two separate aspect of the invention. The patient to be treated is preferably a mammal, in particular a human being.

In one embodiment, the invention relates to 4-[2-(4-methylphenylsulfanyl)-phenyl]piperidine and acid additions salts thereof (compound I) for use in the treatment of ADHD

The compounds of the present invention may be administered alone as a pure compound or in combination with pharmaceutically acceptable carriers or excipients, in either single or multiple doses. The pharmaceutical compositions according to the invention may be formulated with pharmaceutically acceptable carriers or diluents as well as any other known adjuvants and excipients in accordance with conventional techniques such as those disclosed in Remington: The Science and Practice of Pharmacy, 19 Edition, Gennaro, Ed., Mack Publishing Co., Easton, PA, 1995.

The pharmaceutical compositions may be specifically formulated for administration by any suitable route such as the oral, rectal, nasal, pulmonary, topical (including buccal and sublingual), transdermal, intracisternal, intraperitoneal, vaginal and parenteral (including subcutaneous, intramuscular, intrathecal, intravenous and intradermal) route, the oral route being preferred. It will be appreciated that the preferred route will depend on the general condition and age of the subject to be treated, the nature of the condition to be treated and the active ingredient chosen.

Pharmaceutical compositions for oral administration include solid dosage forms such as capsules, tablets, dragees, pills, lozenges, powders and granules. Where appropriate, they can be prepared with coatings.

Liquid dosage forms for oral administration include solutions, emulsions, suspensions, syrups and elixirs.

Pharmaceutical compositions for parenteral administration include sterile aqueous and nonaqueous injectable solutions, dispersions, suspensions or emulsions as well as sterile powders to be reconstituted in sterile injectable solutions or dispersions prior to use.

Other suitable administration forms include suppositories, sprays, ointments, cremes, gels, inhalants, dermal patches, implants, etc.

Conveniently, the compounds of the invention are administered in a unit dosage form containing said compounds in an amount of about 0.1 to 50 mg, such as 1 mg, 5 mg 10 mg, 15 mg, 20 mg, 25 mg, 30 or 35 mg of compound I.

For parenteral routes such as intravenous, intrathecal, intramuscular and similar administration, typically doses are in the order of about half the dose employed for oral administration.

For parenteral administration, solutions of the compound of the invention in sterile aqueous solution, aqueous propylene glycol, aqueous vitamin E or sesame or peanut oil may be employed. Such aqueous solutions should be suitably buffered if necessary and the liquid diluent first rendered isotonic with sufficient saline or glucose. The aqueous solutions are particularly suitable for intravenous, intramuscular, subcutaneous and intraperitoneal administration. The sterile aqueous media employed are all readily available by standard techniques known to those skilled in the art.

Suitable pharmaceutical carriers include inert solid diluents or fillers, sterile aqueous solution and various organic solvents. Examples of solid carriers are lactose, terra alba, sucrose, cyclodextrin, talc, gelatine, agar, pectin, acacia, magnesium stearate, stearic acid and lower alkyl ethers of cellulose. Examples of liquid carriers are syrup, peanut oil, olive oil, phospho lipids, fatty acids, fatty acid amines, polyoxyethylene and water. The pharmaceutical compositions formed by combining the compound of the invention and the pharmaceutical acceptable carriers are then readily administered in a variety of dosage forms suitable for the disclosed routes of administration.

Formulations of the present invention suitable for oral administration may be presented as discrete units such as capsules or tablets, each containing a predetermined amount of the active ingredient, and which may include a suitable excipient. Furthermore, the orally available formulations may be in the form of a powder or granules, a solution or suspension in an aqueous or non-aqueous liquid, or an oil-in-water or water-in-oil liquid emulsion.

If a solid carrier is used for oral administration, the preparation may be tablet, e.g. placed in a hard gelatine capsule in powder or pellet form or in the form of a troche or lozenge. The amount of solid carrier may vary but will usually be from about 25 mg to about 1 g.

If a liquid carrier is used, the preparation may be in the form of a syrup, emulsion, soft gelatine capsule or sterile injectable liquid such as an aqueous or non-aqueous liquid suspension or solution.

Tablets may be prepared by mixing the active ingredient with ordinary adjuvants and/or diluents followed by the compression of the mixture in a conventional tabletting machine. Examples of adjuvants or diluents comprise: Corn starch, potato starch, talcum, magnesium stearate, gelatine, lactose, gums, and the like. Any other adjuvants or additives usually used for such purposes such as colourings, flavourings, preservatives etc. may be used provided that they are compatible with the active ingredients.

Capsules comprising a compound of the present invention may be prepared by mixing a powder comprising said compound with microcrystalline cellulose and magnesium stearate and place said powder in a hard gelatine capsule. Optionally, said capsule may be coloured by means of a suitable pigment. Typically, capsules will comprise 0.25-20% of a compound of the present invention, such as 0.5-1.0%, 3.0-4.0%, 14.0-16.0% of a compound of the present invention. These strengths can be used to conveniently deliver 1, 5, 10, 15, 20 and 25 mg of a compound of the present invention in a unit dosage form.

Solutions for injections may be prepared by dissolving the active ingredient and possible additives in a part of the solvent for injection, preferably sterile water, adjusting the solution to the desired volume, sterilising the solution and filling it in suitable ampoules or vials. Any suitable additive conventionally used in the art may be added, such as tonicity agents, preservatives, antioxidants, etc.

Compound I may either be administered alone or in combination with another therapeutically active compound, wherein the two compounds may either be administered simultaneously or sequentially. Examples of therapeutically active compounds which may advantageously be combined with compound I include sedatives or hypnotics, such as benzodiazepines; anticonvulsants, such as lamotrigine, valproic acid, topiramate, gabapentin, carbamazepine; mood stabilizers such as lithium; dopaminergic drugs, such as dopamine agonists and L-Dopa; drugs to treat ADHD, such as atomoxetine; psychostimulants, such as modafinil, ketamine, methylphenidate and amphetamine; other antidepressants, such as mirtazapine, mianserin and buproprion; hormones, such as T3, estrogen, DHEA and testosterone; atypical antipsychotics, such as olanzapine and aripiprazole; typical antipsychotics, such as haloperidol; drugs to treat Alzheimer's diseases, such as cholinesterase inhibitors and memantine, folate; S-Adenosyl-Methionine; immunmodulators, such as interferons; opiates, such as buprenorphins; angiotensin II receptor 1 antagonists (ATI antagonists); ACE inhibitors; statins; and alphal adrenergic antagonist, such as prazosin.

Compound I may be prepared as outlined in WO 2003/029232 or in WO 2007/144006. Different salts may be achieved by addition of an appropriate acid to the free base followed by precipitation. Precipitation may be brought about by e.g. cooling, removal of solvent, addition of another solvent or a mixture thereof.

The use of the terms "a" and "an" and "the" and similar referents in the context of describing the invention are to be construed to cover both the singular and the plural, unless otherwise indicated herein or clearly contradicted by context. For example, the phrase "the compound" is to be understood as referring to various "compounds" of the invention or particular described aspect, unless otherwise indicated.

Unless otherwise indicated, all exact values provided herein are representative of corresponding approximate values (e.g., all exact exemplary values provided with respect to a particular factor or measurement can be considered to also provide a corresponding approximate measurement, modified by "about," where appropriate).

The description herein of any aspect or aspect of the invention using terms such as "comprising", "having," "including," or "containing" with reference to an element or elements is intended to provide support for a similar aspect or aspect of the invention that "consists of", "consists essentially of', or "substantially comprises" that particular element or elements, unless otherwise stated or clearly contradicted by context (e.g., a composition described herein as comprising a particular element should be understood as also describing a composition consisting of that element, unless otherwise stated or clearly contradicted by context).

### Examples

### Analytical methods

X-Ray powder diffractograms (XRPD) were measured on a PANalytical X'Pert PRO X-Ray Diffractometer using CuK_{α1} radiation. The samples were measured in reflection mode in the 20-range 5-40° using an X'celerator detector.

Elemental composition (CHN) was measured on an Elementar Vario EL instrument from Elementar. About 4 mg of sample was used for each measurement, and the results are given as mean values of two measurements.

### Example 1a HBr salt of compound I

To 442 grams of stirred and slightly heated (approx. 45 °C) 4-(2-p-Tolylsulfanyl-phenyl)-piperidine-1-carboxylic acid ethyl ester as an oil was added 545 ml of 33 wt-% HBr in AcOH (5.7 M, 2.5 eqv.). This mixing gives a 10 °C exotherm. After final addition the reaction mixture is heated to 80 °C and left for 18 hours. A sample is withdrawn and analysed by HPLC and if not completed more 33 wt-% HBr in AcOH must be added. Otherwise the mixture is cooled to 25 °C making the product 4-(2-p-Tolylsulfanyl-phenyl)-piperidine hydrobromide to precipitate. After one hour at 25 °C the thick suspension is added 800 ml diethylether. Stirring is continued for another hour before the product is isolated by filtration, washed with 400 ml diethylether and dried in vacuum at 40 °C overnight. The hydrobromide of compound I was isolated as white solid.

### Example 1b HBr salt of compound I

### 2-(4-tolylsulfanyl)-phenyl bromide

In a stirred nitrogen covered reactor N-methyl-pyrrolidone, NMP (4.5L) was flushed with nitrogen for 20 minutes. 4-Methylbenzenethiol (900g, 7.25mol) was added and then 1,2-dibromobenzene (1709g, 7.25mol). Potassium tert-butoxide (813g, 7.25mol) was finally added as the last reactant. The reaction was exothermic giving a temperature rise of the reaction mixture to 70°C. The reaction mixture was then heated to 120°C for 2 - 3 hours. The reaction mixture was cooled to room temperature. Ethyl acetate (4L) was added and aqueous sodium chloride solution (15%, 2.5L). The mixture was stirred for 20 minutes. The aqueous phase was separated and extracted with another portion of ethyl acetate (2L). The aqueous phase was separated and the organic phases were combined and washed with sodium chloride solution (15%, 2.5L) The organic phase was separated, dried with sodium sulphate and evaporated at reduced pressure to a red oil which contains 20 - 30% NMP. The oil was diluted to twice the volume with methanol and the mixture was refluxed. More methanol was added until a clear red solution was obtained. The solution was cooled slowly to room temperature while seeded. The product crystallises as off white crystals, they were isolated by filtration and washed with methanol and dried at 40°C in a vacuum oven until constant weight.

### Ethyl 4-hydroxy-4-(2-(4-tolylsulfanyl)phenyl)-piperidin-1-carboxylate

In a stirred reactor under nitrogen cover 2-(4-tolylsulfanyl)-phenyl bromide (600g, 2.15ml) was suspended in heptane (4.5L). At room temperature 10M BuLi in hexane (235mL, 2.36mol) was added over 10 minutes. Only a small exotherm was noticed. The suspension was stirred for 1 hour at ambient temperature and then cooled down to -40°C. 1-Carbethoxy-4-piperidone (368g, 2.15 mol) dissolved in THF (1.5L) was added at a rate not faster than the reaction temperature was kept below -40°C. When the reaction has gone to completion, it was warmed to 0°C and 1M HCl (1L) was added keeping the temperature below 10°C. The acid aqueous phase was separated and extracted with ethyl acetate (1L). The organic phases were combined and extracted with sodium chloride solution (15%, 1L). The organic phase was dried over sodium sulphate and evaporated to a semi crystalline mass. It was slurried with ethyl ether (250 mL) and filtered off. Dried in an vacuum oven at 40°C until constant weight.

### Ethyl 4-(2-(4-tolylsulfanyl)phenyl)-piperidin-1-carboxylate

Trifluoroacetic acid (2.8kg, 24.9mol) and triethylsilane (362g, 3.1mol) was charged in a reactor with an efficient stirrer. Ethyl 4-hydroxy-4-(2-(4-tolylsulfanyl)phenyl)-piperidin-1-carboxylate (462g, 1.24mol) was added via a powder funnel in portions.

The reaction was slightly exothermic. The temperature rose to 50°C. After the addition was finalised the reaction mixture was warmed to 60°C for 18 hours. The reaction mixture was cooled down to room temperature. Toluene (750mL) and water (750mL) was added. The organic phase was isolated and the aqueous phase was extracted with another portion of toluene (750mL). The organic phases were combined and washed with sodium chloride solution (15%, 500mL) and dried over sodium sulphate. The sodium sulphate was filtered off, the filtrate evaporated at reduced pressure to a red oil which was processed further in the next step.

### 4-(2-(4-tolylsulfanyl)phenyl)-piperidin hydrobromide

The crude ethyl 4-(2-(4-tolylsulfanyl)phenyl)-piperidin-1-carboxylate as a red oil from example 3 was mixed in a stirred reactor with hydrobromic acid in acetic acid (40%, 545mL, 3.11mol). The mixture was heated at 80°C for 18 hours. The reaction mixture was cooled down to room temperature. During the cooling the product crystallises out. After 1 hour at room temperature ethyl ether (800mL) was added to the reaction mixture, and the mixture was stirred for another hour. The product was filtered off, washed with ethyl ether and dried in a vacuum oven at 50°C until constant weight.

### Example 1c Recrystallisation of the HBr salt of compound I

A mixture of 10.0 grams of the HBr salt of compound I, e.g. prepared as above, was heated to reflux in 100 ml H₂O. The mixture became clear and fully dissolved at 80-90 °C. To the clear solution was added 1 gram of charcoal and reflux was continued for 15 minutes before filtered and left to cool spontaneously to room temperature. During the cooling precipitation of white solid took place and the suspension was stirred for 1 hour at room temperature. Filtration and drying in vacuum at 40 °C overnight produced 6.9 grams (69 %) of the HBr acid addition salt of compound I. See Figure 1 for XRPD. Elemental analysis: 3.92%N, 59.36%C, 6.16%H (theory: 3.85%N, 59.34%C, 6.09%H)

### Example 1d Preparation of stock-solutions of free base

A mixture of 500 ml ethyl acetate and 200 ml H₂O was added 50 grams of the HBr salt of compound I producing a two-phased slurry. To this slurry was added approximately 25 ml conc. NaOH that caused formation of a clear two-phased solution (pH was measured to 13-14). The solution was stirred vigorously for 15 minutes and the organic phase was separated. The organic phase was washed with 200 ml H₂O, dried over Na₂SO₄, filtered and evaporated in vacuum at 60 °C producing the free base in 38 grams yield (99 %) as an almost colourless oil.

Dissolving 10 grams of the oil and adjusting the volume to 150 ml using ethyl acetate produced a 0.235 M stock-solution in ethyl acetate from which aliquots of 1.5 ml (100 mg of the free base) was used.

Dissolving 10 grams of the oil and adjusting the volume to 100 ml using 96-vol% EtOH produced a 0.353 M stock-solution in EtOH from which aliquots of 1.0 ml (100 mg of the free base) was used.

### Example 1e Formation of salts using stock-solutions of the free base

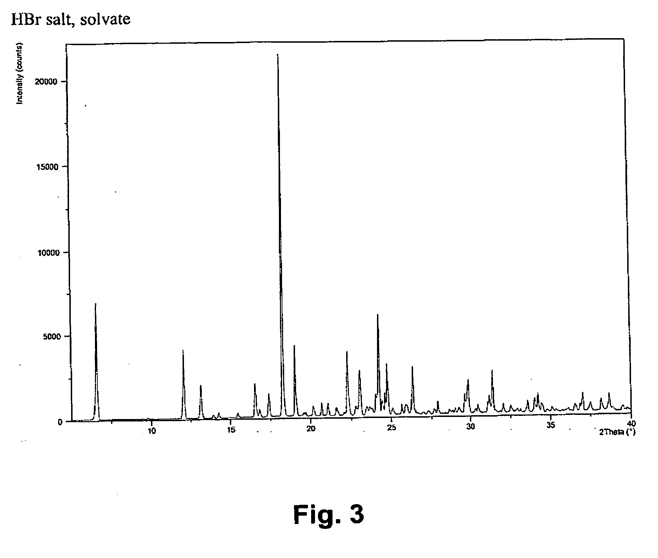

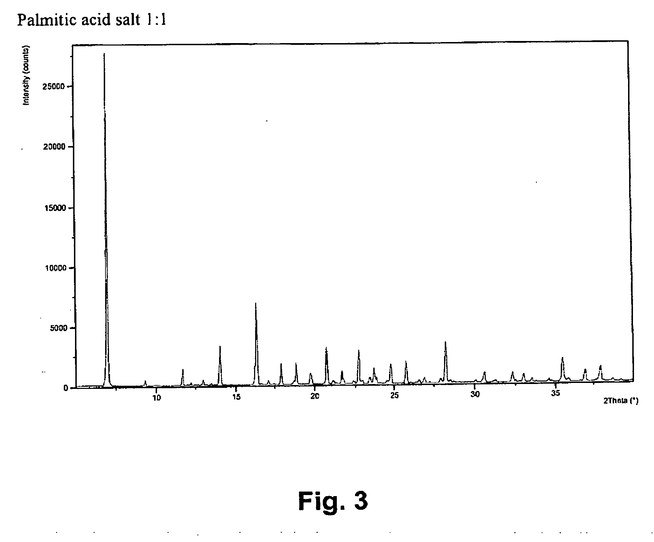

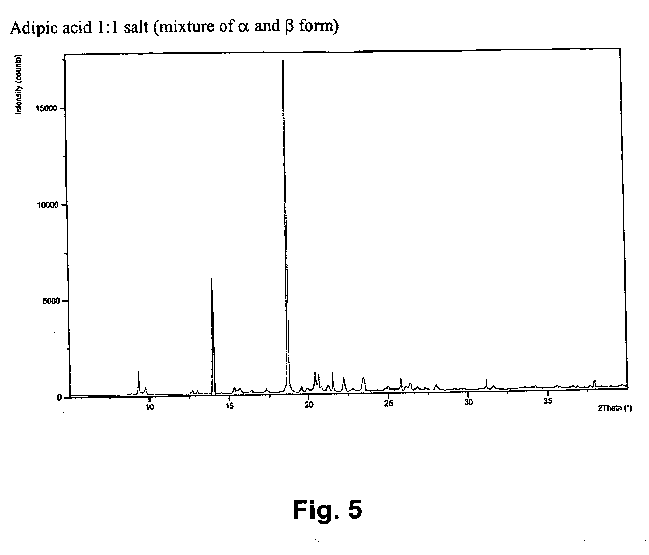

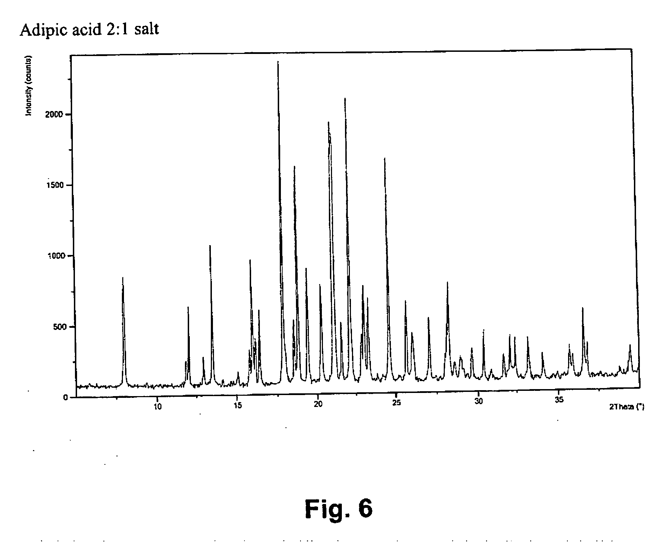

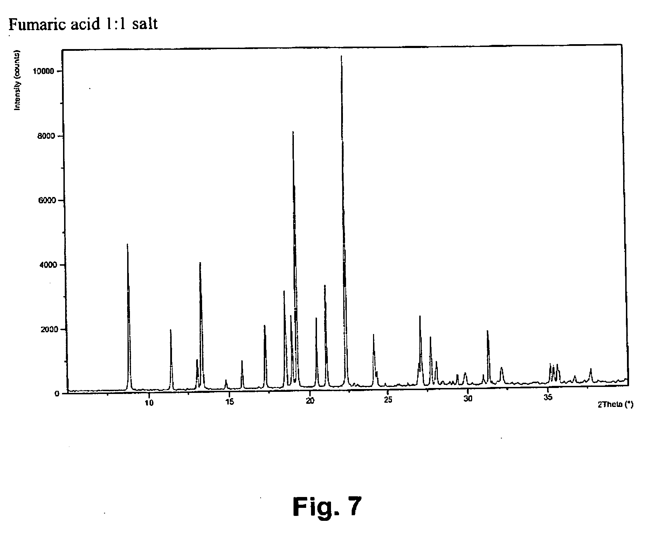

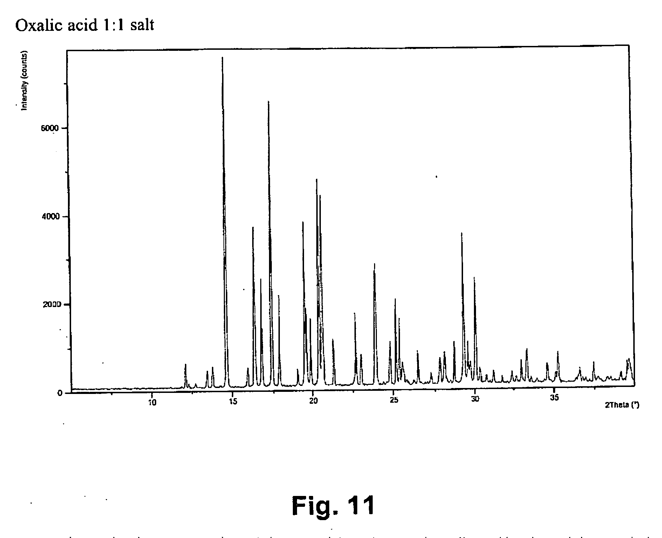

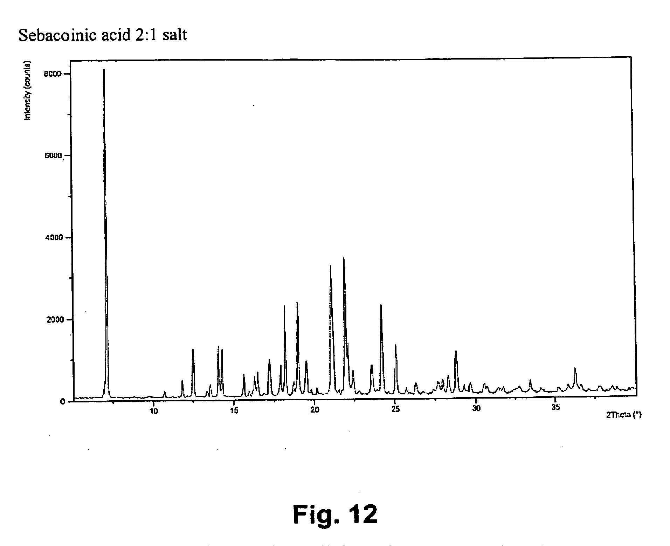

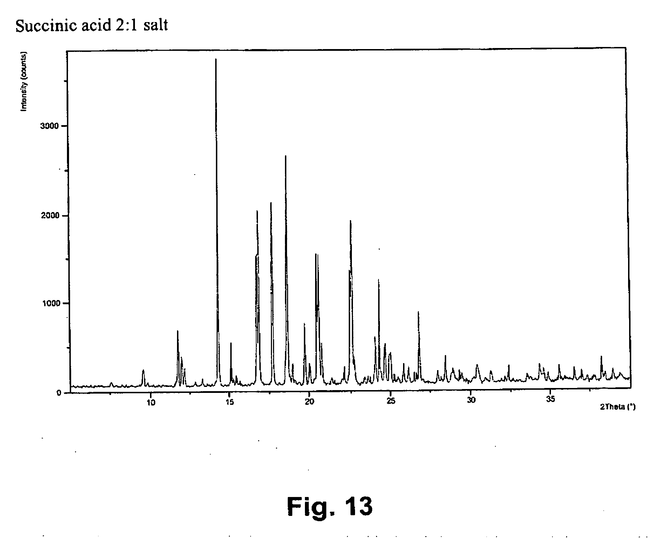

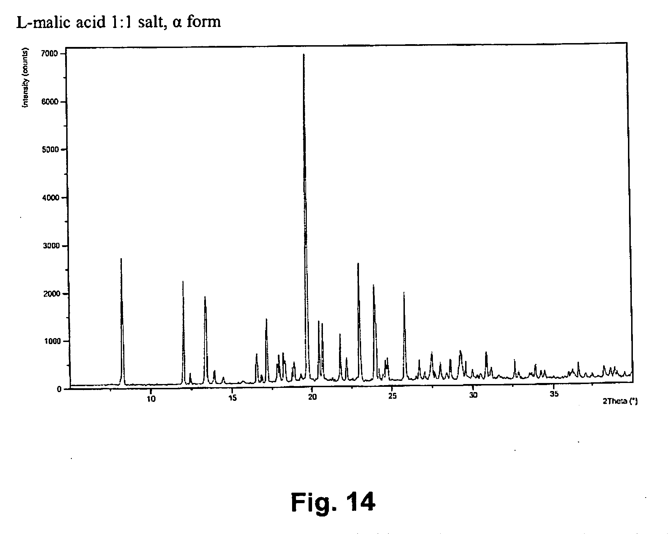

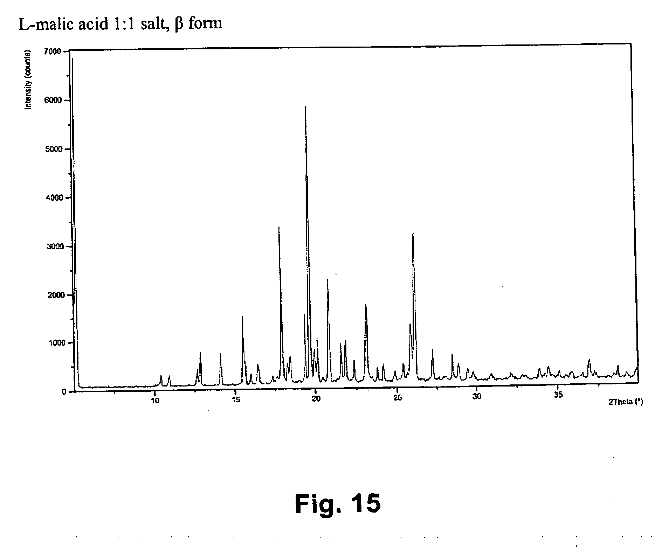

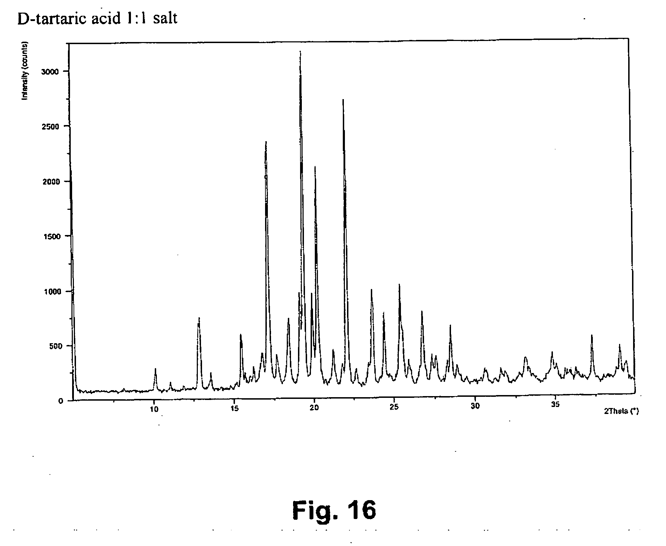

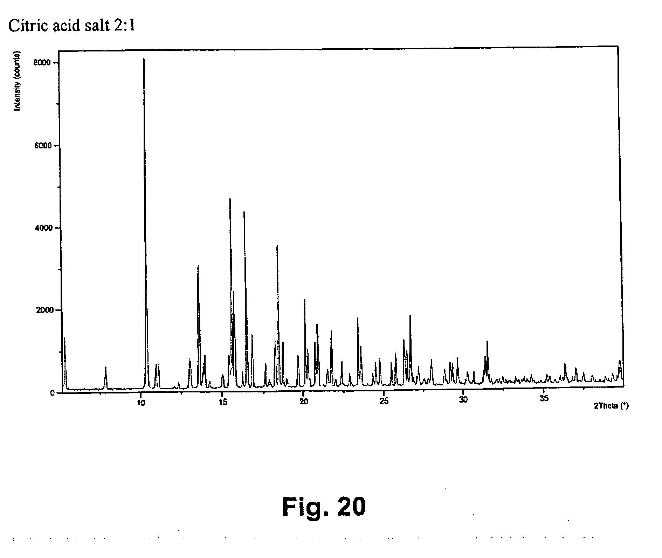

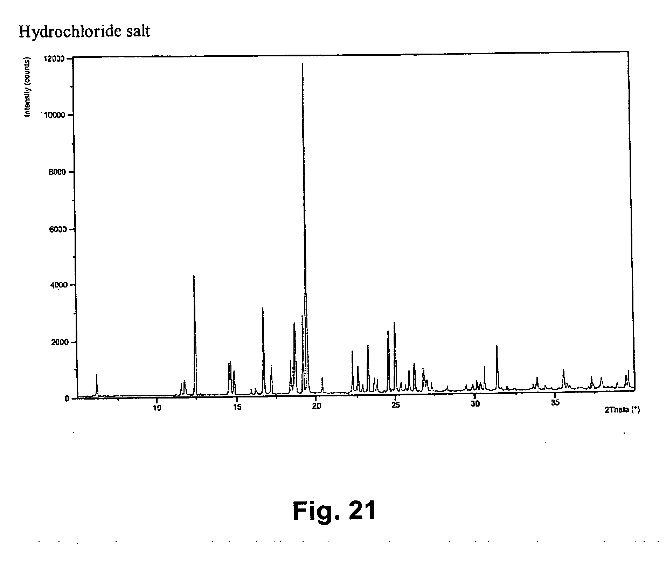

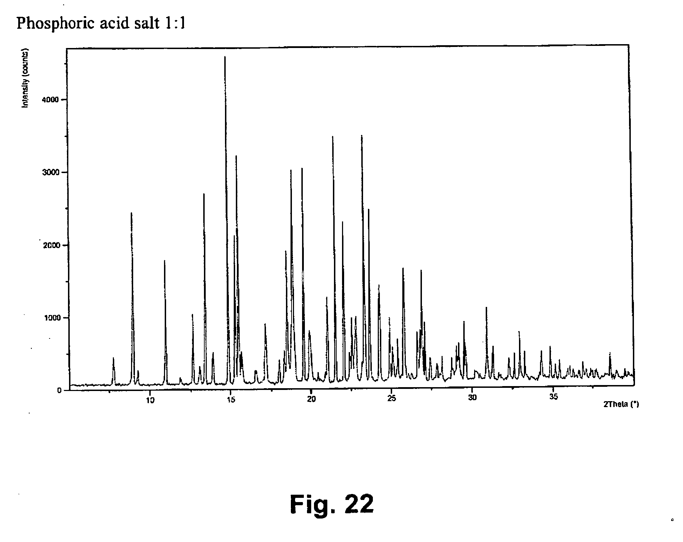

The given aliquots were placed in test tubes and while stirred the appropriate amount of acid was added as indicated in Table 1. If the acid was a liquid it was added neat otherwise it was dissolved in the given solvent prior to addition. After mixing and precipitation stirring was continued overnight and the precipitate collected by filtration. Before drying in vacuum at 30 °C a small reference sample was withdrawn and dried at room temperature without vacuum. This procedure was included in order to test for solvates. Some results are presented in Table 1. XRPD diffractograms are shown in figures 1-22, and selected peak positions are tabulated in Table 2. Table 3 shows the solubilities of compounds of the present invention in water together with pH in the resulting saturated solution. The column "Precipitate" shows whether the precipitate isolated after the solubility determination is identical to the compound dissolved, which is indicative of the formation of hydrates.

**Table 1**

| Acid (Base:Acid) | MW (g/mol) | Amount of Acid (mg or µl) | Solvent | CHN (exp.) | CHN (theory) |
|---|---|---|---|---|---|
| Palmitic acid, hexadecanoic acid) 1:1 | 256.42 | 90.5 | EtOAc | 75.36 9.77 2.46 | 75.64 9.9 2.6 |
| DL-Lactic acid, DL-2-hydroxypropionic acid 1:1 | 90.1 | 31.8 | EtOAc | 66.88 7.26 3.52 | 67.53 729 3.75 |
| Adipicacid, 1,6-hexanedioic acid 1:1 | 146.14 | 51.6 | EtOAc | 66.08 7.23 2.98 | 67.1 7.27 3.26 |
| Adipicacid, 1,6-hexanedioic acid 2:1 | 146.14 | 25.8 | EtOAc | 70.66 7.32 3.82 | 70.75 7.35 3.93 |
| Fumaric acid 1:1 | 116.01 | 40.9 | EtOH | 65.71 6.41 3.35 | 66.14 6.31 3.51 |
| Glutaric acid, 1,5-pentanedioic acid 1:1 | 132.12 | 46.6 | EtOAc | 66.09 6.97 3.2 | 66.48 7.03 3.37 |
| Malonic acid 1:1 | 104.1 | 36.7 | EtOAc | 65.04 6.53 3.54 | 65.09 6.5 3.62 |
| Oxalic acid 1:1 | 90.1 | 31.8 | EtOH | 64.28 6.41 3.61 | 64.32 6.21 3.75 |
| Sebacoinic acid, 1,8-octanedioic acid 2:1 | 202.02 | 35.6 | EtOAc | 71.79 7.86 3.58 | 71.83 7.86 3.64 |
| Succinic acid, 1,4-butanedioic acid, 2:1 | 118.1 | 20.8 | EtOAc | 65.65 6.86 3.4 | 65.80 6.78 3.49 (1:1 salt formed) |
| L-malic acid, L-2-hydroxy butanedioic acid 1:1, α | 134.1 | 47.3 | EtOAc | 62.87 6.20 3.22 | 63.29 6.52 3.36 |
| L-malic acid, L-2-hydroxy butanedioic acid 1:1, β | 134.1 | 47.3 | EtOH | 62.99 6.66 3.13 | 63.29 6.52 3.36 |
| D-tartaric acid, D-2,3-dihydroxy butanedioic acid 1:1 | 150.1 | 53.0 | EtOH | 60.67 6.4 3.07 | 60.95 6.28 3.23 |
| L-aspartic acid 1:1 | 133.1 | 47.0 | EtOH | 59.31 6.7 7.1 (contains excess of acid) | 63.43 6.78 6.73 |
| Glutamic acid 1:1 | 165.15 | 58.3 | EtOH | 56.38 6.88 7.35 (contains excess of acid) | 56.46 6.94 7.06 (for 1:1-salt and acid-monohydrate 1:1) |
| Citric acid 2:1 | 192.13 | 33.9 | EtOAc | 65.93 6.72 3.44 | 66.46 6.64 3.69 |
| HCl/Et₂O 1:1 | 2M | 176.4 | EtOH | | |
| Phosphoric acid 1:1 | 14.7 M | 24.0 | EtOAc | 55.79 6.47 3.43 | 56.68 6.34 3.67 |

**Table 2: Selected X-ray peak positions (°2θ), 2:1 means 2 bases to 1 acid. All values +-0.1°**

| | | | | |
|---|---|---|---|---|
| | | | | |
| Palmitate | 7.00 | 16.34 | 22.73 | 28.21 |
| Stearate | 6.70 | 15.52 | 21.81 | 28.91 |
| Lactate | 5.30 | 8.18 | 9.44 | 17.24 |
| Lactate hydrate | 11.67 | 16.70 | 18.25 | 21.76 |
| hydroxyl-isobutyrate | 5.09 | 16.60 | 20.38 | 27.37 |
| Sebacoin acid salt | 7.18 | 12.53 | 21.11 | 24.19 |
| Adipinic acid salt 2:1 | 8.03 | 13.52 | 17.90 | 24.60 |
| Adipinic acid salt 1:1 α | 9.33 | 14.01 | 18.72 | 20.63 |
| Adipinic acid salt 1:1 β | 15.69 | 21.53 | 25.81 | 31.18 |
| Glutarate 1:1 | 9.39 | 11.70 | 14.05 | 14.58 |
| Succinate 1:1 | 11.74 | 14.33 | 17.75 | 26.84 |
| Fumarate 1:1 | 8.90 | 11.47 | 19.25 | 22.33 |
| Fumarate 2:1 | 8.49 | 12.48 | 17.78 | 23.97 |
| Maleate 1:1 | 12.11 | 15.51 | 17.48 | 22.53 |
| Maleate 1:1 hydrate | 12.81 | 18.76 | 20.53 | 27.31 |
| Malonate α | 10.77 | 16.70 | 19.93 | 24.01 |
| Malonate β | 6.08 | 10.11 | 18.25 | 20.26 |
| Aspanate | 11.05 | 20.1 | 20.60 | 25.00 |
| Aspartate hydrate | 7.80 | 13.80 | 14.10 | 19.63 |
| Glutamate | 7.71 | 14.01 | 19.26 | 22.57 |
| Oxalate | 14.68 | 17.45 | 19.50 | 23.90 |
| Malate 1:1 α | 8.30 | 12.04 | 17.23 | 20.67 |
| Malate 1:1 β | 10.91 | 12.87 | 14.14 | 26.16 |
| Malate hydrate | 12.30 | 15.56 | 19.56 | 23.30 |
| D-tartrate (from EtOH) | 5.08 | 17.18 | 19.42 | 22.10 |
| Hydrochloride | 12.44 | 16.72 | 19.45 | 25.02 |
| Hydrobromide | 6.08 | 14.81 | 19.26 | 25.38 |
| Hydrobromide 1-PcOH solvate | 6.57 | 13.12 | 19.07 | 24.77 |

**Table 3**

| Acid (Base:Acid) | Solubility (mg/ml) | Resulting pH | Precipitate |
|---|---|---|---|
| Palmitic acid, hexadecanoic acid 1:1 | 0.4 | 8.6 | =Start |
| DL-Lactic acid, DL-2-hydroxypropionic acid 1:1 | >150 | 6.1 | =start (after evaporation) |
| Adipicacid, 1,6-hexanedioic acid 1:1 | 2.5 | 4.0 | Partly 2:1 salt |
| Adipicacid, 1,6-hexanedioic acid 2:1 | 1.0 | 7.8 | =start |
| Fumaric acid 1:1 | 0.2 | 3.3 | =start |
| Glutamic acid, 1,5-pentanedioic acid 1:1 | 13 | 4.6 | =start |
| Malonic acid 1: 1(α) | 5.2 | 4.0 | =new form (β) |
| Oxalic acid 1:1 | 1.1 | 2.7 | =Start |
| Sebacoinic acid, 1,8-octanedioic acid 2:1 | 0.7 | 5.5 | =Start |
| Succinic acid, 1,4-butanedioic acid, 2:1 | 2.0 | 4.0 | Hydrate |
| L-malic acid, L-2-hydroxy butanedioic acid 1:1 , β | 2.8 | 4.0 | Hydrate |
| D-tartaric acid, D-2,3-dihydroxy butanedioic acid 1: 1 | 1.8 | 3.5 | Hydrate |
| L-aspartic acid 1:1 | 39 | 4.3 | Hydrate |
| Glutamic acid 1:1 | >35 | 4.6 | - |
| Citric acid 2:1 | 0.5 | 4.7 | =Start |
| Phosphoric acid 1.1 | 6.0 | 2.0 | ? |
| HCl | 4.5 | 6.8 | =Start |
| HBr | 2.4 | 7.0 | =Start |

### Example 2A Serotonin (5-HT) and norepinephrine (NE) reuptake inhibition

Aliquots of test compound and rat cortical synaptosome preparation were preincubated for 10 min/37°C, and then added [³H]NE or [³H]5-HT (final concentration 10 nM). Non-specific uptake was determined in the presence of 10µM talsupram or citalopram and the total uptake was determined in the presence of buffer. Aliquots were incubated for 15 minutes at 37 °C. After the incubation [³H]NE or [³H]5-HT taken up by synaptosomes was separated by filtration through Unifilter GF/C, presoaked in 0.1% PEI for 30 minutes, using a Tomtec Cell Harvester program. Filters were washed and counted in a Wallac MicroBeta counter.

At NET compound I displays an IC₅₀ value of 23 nM. At SERT compound I displays an IC₅₀ value of 8 nM.

### Example 2B 5-HT_{2A} antagonism

Compound I was tested for affinities towards serotonin receptors and was found to exhibit an antagonistic profile with affinity at 5-HT_{2A} receptors (Kᵢ 54 nM). The affinity is calculated from Y = 100/(1+ 10^{(X-logIC}₅₀⁾) where Y denotes % binding and X denotes the concentration of compound. 5 concentrations of compound (1, 10, 30, 100, 1000 nM) were used to calculate the IC50 value. Ki was calculated from the Cheng Prusoff equation Ki = (IC₅₀/(1+ ([L]/Kd)) Afftiny was determined at MDL Pharmaservices catalogue number 271650.

In mammalian cells expressing human 5-HT_{2A} receptors compound I displays competitive antagonistic properties. The compounds bind to 5-HT_{2A} receptors with a Ki of < 100 nM and in a functional assay the compounds antagonise 5-HT evoked release of Ca²⁺ from intracellular stores with a Kb of 67 nM. A schild analysis revealed competitive antagonism with a Kb of 100 nM.

The experiment was carried out as follows. 2 or 3 days before the experiment CHO cells expressing 250 fmol/mg human 5-HT_{2A} receptors are plated at a density sufficient to yield a mono-confluent layer on the day of the experiment. The cells are dye loaded (Ca²⁺-kit from Molecular Devices) for 60 minutes at 37° C in a 5% CO₂ incubator at 95% humidity. Basal fluorescence was monitored in a fluorometric imaging plate reader or FLIPR ³⁸⁴ from Molecular Devices (Sunnyvale, CA) with an excitation wavelength of 488 nm and an emission range of 500 to 560 nm. Lacer intensity was set to a suitable level to obtain basal values of approximately 8000-10000 fluorescence units. The variation in basal fluorescence should be less than 10%. EC₅₀ values are assessed using increasing concentrations of test compound covering at least 3 decades. pA2 values are assessed challenging full dose response curves of 5-HT with four different concentrations of compound (150, 400 1500 and 4000 nM). Kb values were also assessed challenging 2 decades of concentrations of test substances with EC₈₅ of 5-HT. Test substances are added to the cells 5 minutes before the 5-HT. Kᵢ values are calculated using Cheng-Prusoff equation.

### Example 2C 5-HT_{3A} receptor antagonism

In oocytes expressing human-homomeric 5-HT_{3A} receptors 5-HT activates currents with an EC₅₀ of 2600 nM. This current can be antagonised with classical 5-HT₃ antagonists such as ondansetron. Ondansetron displays a Ki value below 1 nM in this system. Compounds of the present invention exhibit potent antagonism in low concentrations (0.1 nM - 100 nM) (IC₅₀ ~ 10 nM/ Kb ~ 2 nM) and agonistic properties when applied in higher concentrations (100 - 100000 nM) (EC₅₀ ~ 2600 nM) reaching a maximal current of approximately 70-80 % of the maximal current elicited by 5-HT itself. In oocytes expressing rat-homomeric 5-HT_{3A} receptors 5-HT activates currents with an EC₅₀ of 3.3 µM. The experiments were carried out as follows. Oocytes were surgically removed from mature female *Xenepus laevis* anaesthetized in 0.4 % MS-222 for 10 - 15 min. The oocytes were then digested at room temperature for 2-3 hours with 0.5 mg/ml collagenase (type IA Sigma-Aldrich) in OR2 buffer (82.5 mN NaCl, 2.0 mM KCI, 1.0 mM MgCl2 and 5.0 mM HEPES, pH 7.6). Oocytes avoid of the follicle layer were selected and incubated for 24 hours in Modified Barth's Saline buffer [88 mM NaCl, 1 mM KCI, 15 mM HEPES, 2.4 mM NaHCO₃, 0.41 mM CaCl₂, 0.82 mM MgSO₄, 0.3 mM Ca(NO₃)₂] supplemented with 2 mM sodium pyruvate, 0.1 U/l penicillin and 0.1 µg/l streptomycin. Stage IV-IV oocytes were identified and injected with 12-48 nl of nuclease free water containing 14 - 50 pg of cRNA coding for human 5-HT3A receptors receptors and incubated at 18°C until they were used for electrophysiological recordings (1 - 7 days after injection). Oocytes with expression of human 5-HT3 receptors were placed in a 1 ml bath and perfused with Ringer buffer (115 mM NaCl, 2.5 mM KCI, 10 mM HEPES, 1.8 mM CaCl₂, 0.1 mM MgCl₂, pH 7.5). Cells were impaled with agar plugged 0.5-1 MΩ electrodes containing 3 M KCl and voltage clamped at -90 mV by a GeneClamp 500B amplifier. The oocytes were continuously perfused with Ringer buffer and the drugs were applied in the perfusate. 5-HT agonist-solutions were applied for 10 - 30 sec. The potencies of 5-HT₃ receptor antagonists were examined by measuring concentration-response against 10 µM 5-HT stimulation.

### Example 2D α_{1A}, receptor antagonism

Compound I was tested for affinities towards the α_{1A} receptor and was found to exhibit an antagonistic profile with medium affinity for α_{1A} receptors (Ki = 34 nM).

On the day of the experiments membranes (see below for description of membrane preparation) are thawed and homogenized in buffer using an ultra turrax and diluted to the desired concentration (5 µg / well - 5 µg / 900 µl, store on ice until use).

The experiment is initiated by mixing of 50 µl test compound, 50µl [³H]-Prazosin and 900µl membranes, and the mixture is incubated for 20 minutes at 25 °C. Non-specific binding is determined in the presence of 10µM WB-4101 and the total binding is determined in the presence of buffer. After the incubation, bound ligand is separated from unbound by filtration through Unifilter GFB, presoaked in 0.1 % PEI for 30 minutes, using a Tomtec Cell Harvester program (D4.2..4). 96 well. Filters are washed 3 times with 1 ml ice-cold buffer, dried at 50 °C and 35µl scintillation liquid/well is added to the filters. Bound radioactivity is counted in a Wallac OY 1450 MicroBeta. The affinity is calculated from Y = 100/(1+ 10^{(X-logIC}₅₀⁾) where Y denotes % binding and X denotes the concentration of compound. Concentrations of compound covering 2 decades were used to calculate the IC50 value. Ki was calculated from the Cheng Prusoff equation Ki = (IC₅₀/(1+ ([L]/Kd))

In a functional assay compound I antagonises adrenaline evoked release of Ca²⁺ from intracellular stores and a functional assay revealed that compounds were antagonists.

These experiments were carried out essentially as described below.

All cells were cultured in DMEM medium supplemented with 10% BCS, 4 mM L-glutamine (or 2 mM in the case of COS-7), and 100 units/ml penicillin plus 100 µg/ml streptomycin, at 37 oC, in 5% CO2.

Twenty-four hours prior to assays, CHO cells expressing the human alpha_{1A-7} receptors were seeded into 384-well black wall microtiter plates coated with poly-D-lysine. Culture medium was aspirated and cells were dye-loaded with 1.5 µM Fluo-4 in assay buffer composed of Hank's Balanced Salt Solution (138 mM NaCl, 5 mM KCl, 1.3 mM CaCl₂, 0.5 mM MgCl₂, 0.4 mM MgSO₄, 0.3 mM KH₂PO₄, 0.3 mM Na₂HPO₄, 5.6 mM glucose) plus 20 mM HEPES pH 7.4, 0.05% BSA and 2.5 mM probenicid (50 µl/well) for 1 hour in 5% CO₂ at 37 °C. After excess dye was discarded, cells were washed in assay buffer and layered with a final volume equal to 45 µl/well (or 30 ul/well for antagonist assay). In the case of antagonist evaluation, antagonist or vehicle was added at this point as a 15 µl aliquot in 4% DMSO-containing buffer at 4x the final concentration (final DMSO = 1%), followed by a 20 min incubation. Basal fluorescence was monitored in a fluorometric imaging plate reader or FLIPR^{™} from Molecular Devices (Sunnyvale, CA) with an excitation wavelength of 488 nm and an emission range of 500 to 560 nm. Laser excitation energy was adjusted so that basal fluorescence readings were approximately 8,000 relative fluorescent units (RFU). Cells were then stimulated at room temperature with agonists diluted in assay buffer (15 µl), and RFU were measured at 1.5 second intervals over a period of 2.5 min. Maximum change in fluorescence was calculated for each well. Concentration-response curves derived from the maximum change in fluorescence were analyzed by nonlinear regression (Hill equation). For antagonistic determinations, after 20 min of compound incubation (as above), fixed concentrations of standard agonist serotonin were added.

### Example 2E Increase in dopamine

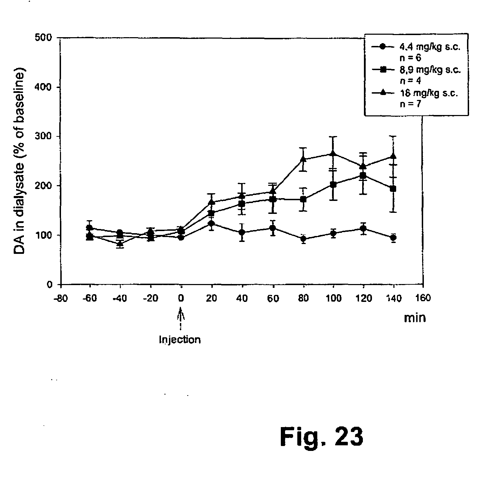

A single injection of compound I dose-dependently increased extracellular DA levels in the rat frontal cortex. The compound of the present invention at 8.9mg/kg and 18mg/kg s.c., enhanced the DA levels by approximately 100% and 150%, respectively, above baseline levels as depicted in figure 23. Amounts are calculated as the free base.

### Method.

Male Sprague-Dawley rats, initially weighing 275-300 g, were used. The animals were housed under a 12-hr light/dark cycle under controlled conditions for regular indoor temperature (21±2°C) and humidity (55±5%) with food and tap water available ad libitum. For the three-day treatment experiments osmotic minipumps (Alzet, 2ML1) were used. The pumps were filled under aseptic conditions and implanted subcutaneously under sevoflurance anaesthesia. The experiments were carried out with the minipumps on board. Blood samples for measuring plasma levels of the test compound after 3 days of treatment were collected at the end of the experiments.

### Surgery and microdialysis experiments.

Animals were anaesthetised with hypnorm/dormicum (2 ml/kg) and intracerebral guide cannulas (CMA/12) were stereotaxically implanted into the hippocampus, positioning the dialysis probe tip in the ventral hippocampus (co-ordinates: 5,6 mm anterior to bregma, lateral -5,0 mm, 7,0 mm ventral to dura or in the frontal cortex (co-ordinates: 3,2 mm anterior to bregma; lateral, 3.0 mm; 4,0 mm ventral to dura). Anchor screws and acrylic cement were sued for fixation of the guide cannulas. The body temperature of the animals was monitored by rectal probe and maintained at 37°C. The rats were allowed to recover from surgery for 2 days, housed singly in cages. On the day of the experiment a microdialysis probe (CMA/12, 0,5 mm diameter, 3 mm length) was inserted through the guide cannula. The probes were connected via a dual channel swivel to a microinjection pump. Perfusion of the microdialysis probe with filtered Ringer solution (145 mm NaCl, 3 mM KCI, 1 mM MgCl₂, 1,2 mM CaCl₂) was begun shortly before insertion of the probe into the brain and continued for the duration of the experiment at a constant flow rate of 1 (1,3) µL/min. After 180 min of stabilisation, the experiments were initiated. Dialysates were collected every 20 (30) min.
After the experiments the rats were sacrificed by decapitation, their brains removed, frozen and sliced for probe placement verification.

### Analysis of dialysates.

Concentration of dopamine in the dialysates was analysed by means of HPLC with electrochemical detection. The monoamines were separated by reverse phase liquid chromatography (ODS 150 x 3 mm, 3 µM). Dopamine: Mobile phase consisting of 90 mM NaH₂PO₄, 50 mM sodium citrate, 367 mg/l sodium 1-octanesulfonic acid, 50 µM EDTA and 8% acetonitrile (pH 4.0) at a flow rate of 0.5 ml/min. Electrochemical detection was accomplished using a coulometric detector; potential set at 250 mV (guard cell at 350 mV) (Coulochem II, ESA).

### Example 2F Increase in acetylcholin

The experiment was designed to evaluate the effects of compound I on extracellular levels of acetylcholine in the prefrontal cortex of freely-moving rats.

Male Wistar rats (280-350 g; Harlan, Zeist, The Netherlands) were used for the experiments. Rats were individually housed in plastic cages (30 x 30 x 40 cm) and had ad libitum access to food and water.

Rats were anesthetized using isoflurane (2%, 400 mL/min N₂O, 400 ml/min O₂). Lidocain (10 % m/v) was used for local anesthesia. Each animal was placed into a stereotaxic frame (Kopf instruments, USA), and home-made I-shaped probes (Hospal AN 69 membrane, 4 mm exposed surface) were inserted into the medial prefrontal cortex (mPFC) using the rat brain atlas of Paxinos and Watson (1982). Coordinates for the tip of the probe was mPFC [AP = 3.4 mm, L = -0.8 mm, V = 5.0 mm].The probe was then fixed to the skull with dental cement and a srew. Flunixin (1 mg/kg s.c.) was administered as post-operative analgesic.

Experiments were carried out 24-48 hours after surgery. On the day of the experiment, rats were connected with flexible PEEK tubing to microperfusion pumps (CMA 102), and the dialysis probes were perfused with a Ringer buffer containing 147 mM NaCl, 3.0 mM KCI, 1.2 mM CaCl₂, and 1.2 mM MgCl₂, at a flow rate of 1.5 µL/min. Microdialysis samples were collected at 30 min intervals into mini-vials containing 55 µL 0.02 M formic acid for determination of acetylcholine. Samples were collected by an automated fraction collector (CMA 142), and stored at -80° C until analyzed. After completion of the experiments the rats were sacrificed. The brains were removed and cured in paraformaldehyde solution (4% m/v). The positioning of each probe was verified histologically according to Paxinos and Watson (1982), by making coronal sections of the brain.

The test compound was dissolved in 10 % 2-OH-propyl-beta-cyclodextrin and administration occurred by subcutaneous injections of 5 mL/kg volumes in different doses.

Concentrations of acetylcholine were determined by HPLC with tandem mass spectrometry (MS/MS) detection.

Aliquots (25 µL) were injected onto the HPLC column by an automated sample injector (PerkinElmer Instruments, series 200). Chromatographic separation was performed on a reverse-phase 150 x 2.00 mm (4 µm) analytical column (Phenomenex Synergy MAX-RP, Bester) protected by a 4 x 2.0 mm guard column (Phenomenex Synergy MAX-RP AJO-6073, Bester), both held at a temperature of 30° C. The mobile phase (isocratic) consisted of ultrapurified water (UP), acetonitrile (ACN), and trifluoroacetic acid (TFA) (UP:ACN:TFA = 95.0:0.5:0.1 v/v/v%). Mobile phase was run through the system at a flow rate of 0.300 mL/min by an HPLC pump (PerkinElmer Instruments, series 200 micro pump).

The LC/MS analyses were performed using a API 4000 MS/MS system consisting of a API 4000 MS/MS detector and a Turbo Ion Spray interface (both from Applied Biosystems, the Netherlands). The acquisitions were performed in positive ionization mode, with ion spray voltage set at 5.5 kV, the nebulizer gas pressure at 50 psig (on a SCIEX scale 0-90) with a probe temperature of 600°C. The instrument was operated in multiple-reaction-monitoring (MRM) mode for detection of acetylcholine (precursor 146.1 Da, product 86.8 Da). The collision energy was 21.0 eV, and the collision gas (nitrogen) pressure was held at 7 (on a SCIEX scale of 0-12). Data were calibrated and quantitated using the Analyst^{tm} data system (Applied Biosystem, version 1.2).

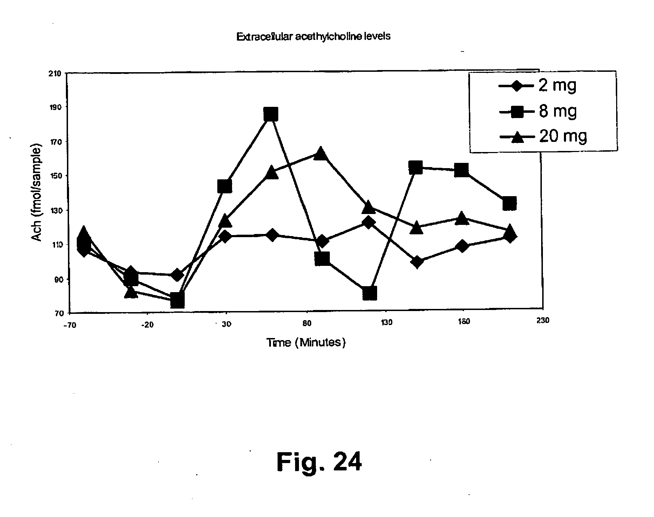

Two consecutive microdialysis samples with less then 50 % variation were taken as baseline levels and set at 100 %. Changes in acetylcholine concentration were expressed as percent of baseline within the same subject.
The data are shown in Figure 24

### Example 2G Increase in acetylcholine

The experiment was designed to evaluate the effects of compound I on extracellular levels of acetylcholine in the prefrontal cortex and ventral hippocampus of freely-moving rats.
Male Sprague-Dawley rats, initially weighing 275-300 g, were used. The animals were housed under a 12-hr light/dark cycle under controlled conditions for regular indoor temperature (21±2°C) and humidity (55±5%) with food and tap water available ad libitum.

### Surgery and microdialysis experiments

Rats were anaesthetised with hypnorm/dormicum (2 ml/kg) and intracerebral guide cannulas (CMA/12) were stereotaxically implanted into the hippocampus, aiming to position the dialysis probe tip in the ventral hippocampus (co-ordinates: 5,6 mm posterior to bregma, lateral -5,0 mm, 7,0 mm ventral to dura or in the frontal cortex (co-ordinates: 3,2 mm anterior to bregma; lateral, 0,8 mm; 4,0 mm ventral to dura). Anchor screws and acrylic cement were used for fixation of the guide cannulas. The body temperature of the animals was monitored by rectal probe and maintained at 37°C. The rats were allowed to recover from surgery for 2 days, housed singly in cages. On the day of the experiment a microdialysis probe (CMA/12, 0,5 mm diameter, 3 mm length) was inserted through the guide cannula.

The probes were connected via a dual channel swivel to a microinjection pump. Perfusion of the microdialysis probe with filtered Ringer solution (145 mm NaCl, 3 mM KCI, 1 mM MgCl₂, 1,2 mM CaCl₂ containing 0.5 µM neostigmine) was begun shortly before insertion of the probe into the brain and continued for the duration of the experiment at a constant flow rate of 1 µl/min. After 180 min of stabilisation, the experiments were initiated. Dialysates were collected every 20 min. After the experiments the animals were sacrificed, their brains removed, frozen and sliced for probe placement verification.

### Analysis of dialysate acetylcholine

Concentration of acetylcholine (ACh) in the dialysates was analysed by means of HPLC with electrochemical detection using a mobile phase consisting of 100 mM disodium hydrogenphosphate, 2.0 mM octane sulfonic acid, 0.5 mM tetramethylammonium chloride and 0.005% MB (ESA), pH 8.0. A pre-column enzyme reactor (ESA) containing immobilised choline oxidase eliminated choline from the injected sample (10 µl) prior to separation of ACh on the analytical column (ESA ACH-250); flow rate 0.35 ml/min, temperature: 35°C. After the analytical column the sample passed through a post-column solid phase reactor (ESA) containing immobilised acetylcholineesterase and choline oxidase. The latter reactor converted ACh to choline and subsequently choline to betaine and H₂O₂. The latter was detected electrochemical by using a platinum electrode (Analytical cell: ESA, model 5040).

### Data presentation

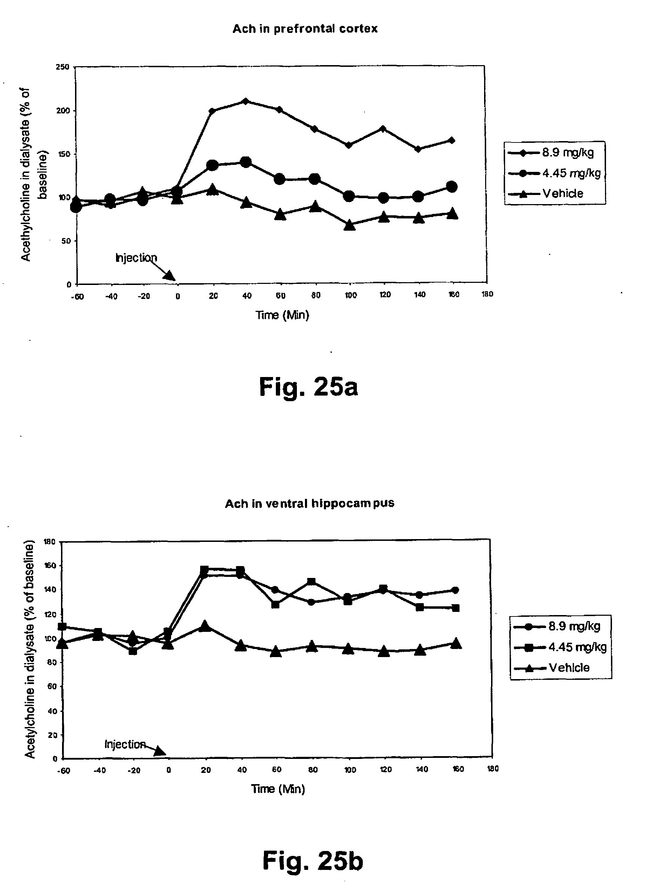

In single injection experiments the mean value of 3 consecutive ACh samples immediately preceding compound administration served as the basal level for each experiment and data were converted to percentage of basal (mean basal pre-injection values normalized to 100%). The data are presented in Figure 25a and 25b.

The data presented in Figure 24 show unexpected drops in the acetycholine levels (see e.g. 8 mg/kg) which are difficult to explain and which are ascribed to experimental uncertainty. Overall, both data sets from example 2F and 2G show the same, i.e. a dose dependent increase in the extra-cellular acetylcholine levels in the brain. This pre-clinical finding is expected to translate into an improvement in cognition in a clinical setting useful e.g. in the treatment of diseases characterised by a cognitive impairment, such as e.g. Alzheimer's patients, partial responders, cognitive impairment etc.

### Example 3 Effects of compound I in Spontaneously hypertensive rats - an animal model of ADHD

Core symptoms of ADHD are attention deficit, hyperactivity and increased impulsiveness. Spontaneously hypertensive rats (SHR) were used as animal model for attention deficit hyperactivity disorder (ADHD), Wistar Kyoto rats (the root strain of SHR) served as controls [Biol Psychiatry. 57, 1239-47, 2005]. To assess these symptoms, an operant task involving a delayed food-reward was used to measure attention- and impulsiveness-related parameters. Attention deficits were measured as increase in the number of lever presses on the wrong side. Impulsiveness was measured as lever presses and reward chamber inspections during the OFF state in test sessions without delayed reward and during the delay interval in the test sessions with delayed rewarding. Hyperactivity was monitored by circadian recording in infra-red cages.

SHR and Wistar Kyoto rats did not markedly differ in acquiring the task. Furthermore, the two vehicle groups of SHR and Wistar Kyoto rats showed the same general motivation of food seeking, reflected by an equal number of rewards obtained. SHR rats exhibited a slight attention deficit compared to Wistar Kyoto rats. Impulsiveness was markedly increased in SHR compared with Wistar Kyoto rats and hyperactivity was observed as well.

Test groups: one group of Wistar Kyoto rats as controls, one group of vehicle-treated SHR (negative control), two groups of intermittently methylphenidate-treated SHR (2 mg/kg and 5 mg/kg i.p, reference groups), one group of SHR treated chronically with methylphenidate via the drinking water (achieved douse: ~10 mg/kg/day) and two groups of SHR intermittently treated with compound I (5 mg/kg and 10 mg/kg free base).

Methods: Operant testing was performed in 20 hours sessions, each in operant behaviour cages with two levers and adjacent reward chambers on the right and left side of the lever panel. The animals had access to food only via lever presses, and fluid was freely available. Training and testing consisted of the following phases:

### Acquisition phase (no treatment)

(1) Both levers were continuously active (indicated by a signal light). Each lever press resulted in an immediate presentation of a reward in the adjacent reward chamber signalled by a single house light.
(2) Like (1), except that only one lever was active at a given time, switching in a five minute rhythm between the left and the right side. Signal light indicated the correct side.
(3) Like (2), except that the lever on the correct side was set inactive every 20 seconds for a period of 20 seconds. The signal light on the correct side indicated the state of the lever (ON or OFF).

### Test phase (during treatment)

(1) Like Acquisition (3)
(2) Like (1), except that after a lever press of an active lever, the reward was not presented immediately, but after a delay interval of 5 seconds. During this time, the according signal light was OFF and the lever was set inactive.
(3) Like (2) but with a delay interval of 10 seconds.
(4) Like (3) but with a delay interval of 20 seconds.

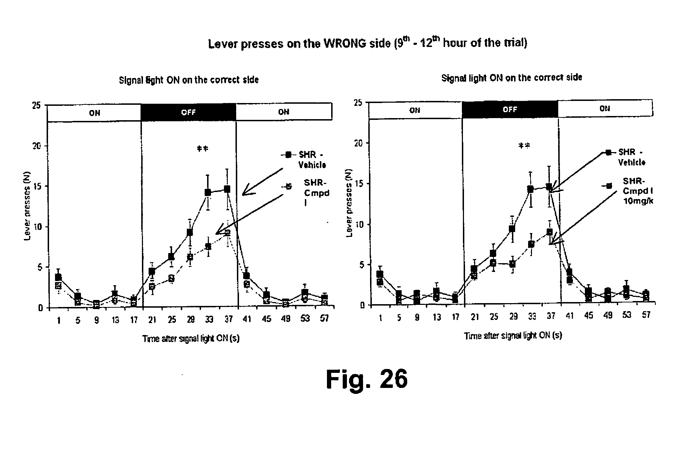

Compound I at both doses tested (5 and 10 mg/kg i.p., injected 30min before testing) had significant effects in SHR rats. These effects did not affect the acquisition of the task or general motivation of food seeking, but attention deficits and impulsiveness were reduced together with hyperactivity - locomotor activity was dose-dependently depressed during circadian activity monitoring for 1 hour without prolonged effects. A representative graph showing the effects of compound I on attention-deficits and impulsivity in SHR rats is provided in figure 26.

Methylphenidate revealed no consistent effects on operant behaviour; there was no reduction of attention deficit or impulsiveness. Intermittent administration of methylphenidate markedly and dose-dependently aggravated the hyperactivity of SHR. The effect lasted a few hours. Chronic administration did not alter the time course of activity.

The results from this model indicate that compound I effects impulsivity and attention by a different mechanism than methylphenidate. The lack of an effect of methylphenidate in this model could be due to the fact that methylphenidate has been shown to be effective in adolescent, but not adult rats [Psychopharmacology (Berl), 193(2), 215-23, 2007].

## Claims

1. 4-[2-(4-methylphenylsulfanyl)phenyl]piperidine 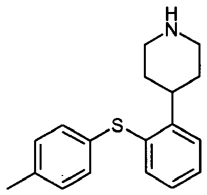 and acid additions salts thereof for use in the treatment of ADHD.

2. The compound for use according to claim 1, which compound is the HBr addition salt.

3. The compound for use according to claim 2, which compound is **characterised by** peaks in an XRPD at approximately 6.08, 14.81, 19.26 and 25.38°2θ.

4. The compound for use according to claim 3, which compound is **characterised by** an XRPD as depicted in figure 1.

5. The compound for use according to any of claims 1-4, wherein said compound is administered at 5-60 mg/day.

## Patentansprüche

1. 4-[2-(4-Methylphenylsulfonyl)phenyl]Piperidin 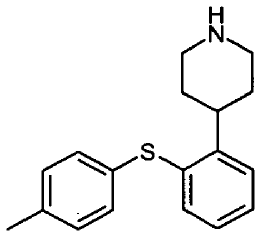 und Säureadditionssalze davon zur Verwendung bei der Behandlung von ADHS.

2. Präparat zur Verwendung nach Anspruch 1, dessen Verbindung im HBr-Additionssalz verwendet wird.

3. Präparat zur Verwendung nach Anspruch 2, dessen Verbindung durch Spitzen in einem XRPD bei ca. 6,08, 14,81, 19,26 und 25,38°2θ gekennzeichnet ist.

4. Präparat zur Verwendung nach Anspruch 3, dessen Verbindung durch XRPD gekennzeichnet ist, wie in Abb. 1 dargestellt.

5. Präparat zur Verwendung nach einem der Ansprüche 1-4, wobei die Verbindung mit 5-60 mg/Tag verabreicht wird.

## Revendications

1. 4-[2-(4-méthylphénylsulfanyl)phényl]pipéridine : 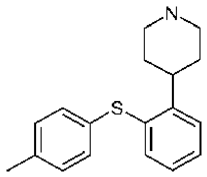 et ses sels d'addition avec des acides pour usage dans le traitement du trouble du déficit de l'attention avec hyperactivité (TDAH).

2. Composé pour usage selon la revendication 1, lequel composé est le sel d'addition avec HBr.

3. Composé pour usage selon la revendication 2, lequel composé est **caractérisé par** des pics dans une XRPD à environ 6,08, 14,81, 19,26 et 25,38° de 2θ.

4. Composé pour usage selon la revendication 3, lequel composé est **caractérisé par** une XRPD comme présenté sur la Fig. 1.

5. Composé pour usage selon l'une quelconque des revendications 1 à 4, dans lequel ledit composé est administré à raison de 5 à 60 mg/jour.
